# EUROPEAN PATENT APPLICATION

(11) **EP 0 654 693 A1**
(43) Date of publication of application: **24.05.1995**
(21) Application number: 94500178.2
(22) Date of filing: 10.11.1994
(51) Int. Cl.: G02C 7/10, A61F 9/02

(54) **Improved structure for protector glasses**

(30) Priority: 24.11.1993 ES 9302469
(71) Applicant: Sanmiguel Acebedo, Cecilia, E-46004 Valencia (ES)
(72) Inventor: Sanmiguel Acebedo, Cecilia, E-46004 Valencia (ES)
(74) Representative: Urizar Anasagasti, José Antonio

(57) **Abstract**

Improved structure for protection glasses, characterised by being glasses with therapeutical purposes in the treatment of ocular diseases where an ocular filter is necessary agains ultraviolet beams, being possible to incorporate in said structure, cristals for the vision of persons who need them due to problems of myopia, hypermetropia, etc.

## Description

The object of the present invention is based on a structure for protection of the eyes in the whole contour of the incidenceof light over the same. For that reason the invention is based on glasses of normal appearance which incorporatea series of elements destined to that end.

Some patients in the period of recovery after certain ocular surgical operations have to avoid direct exposures to the light either solar or artificial. On theother hand, other patients have to avoid certain kinds of determined radiations within the light spectrum. In this manner, there are certain interventions which require avoiding, specifically, any radiation whose wave-length is in a sector of the spectrum such as ultraviolet radiation.

To that end, the process of recovery of a patient after an intervention was characterised either by avoiding the exposure of any kind of radiation to the eye, or wearing glasses specially equipped with filters suitable for the type of radiation to be avoided. These glasses had an essential function which was ocular protection without caring about the aesthetic or the appearance of the glasses. One can thus say that the protecting element was essentailly functional and only destined to thatend, that is, giving freedom to the patient in his process of recovery so that he may carry on a normal life.

The fact of using means of protection included in a structure such glasses, would liberate the patient from the discomfort of having to use bandages which would completely eliminate the visibility.

In this case, the object of the invention has an additional characteristic which would allow for complete freedom of the patient in his process of recovery and continuation during the time indicated by the ophthalmologist which perform his treatment, said characteristic being that of incorporating ultraviolet light filtersto a structure with a completely normal appearance, so that the patient would not be hindered during his normal social life by carrying glasses which include a higher degree of aesthetic appearance to the contrary of the ones known up to now, which due to thier eccentric shape obliged the patient to stay in his usual recovery place during the treatment process.

In order to understand better the object of the present invention a practical and preferred embodiment of the same is represented in the accompanying drawings.

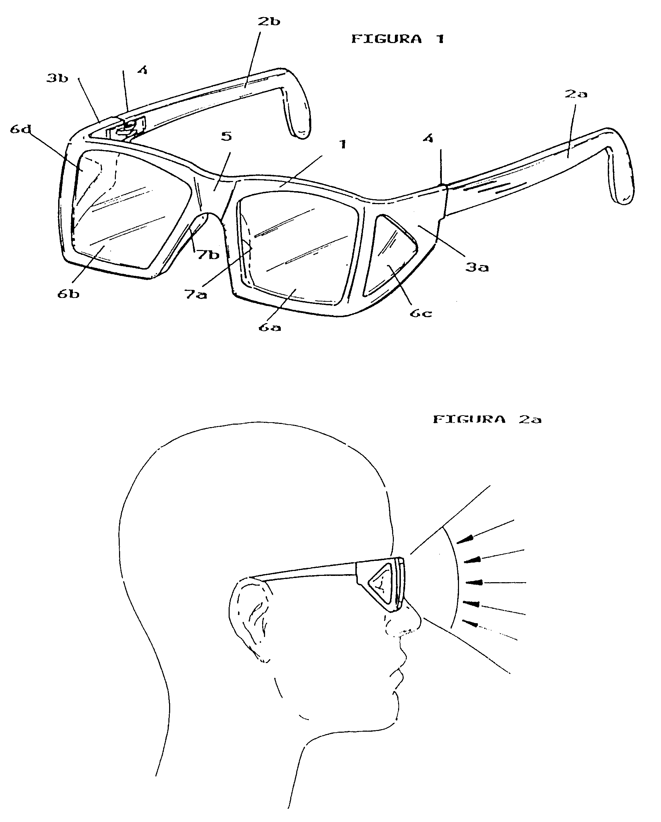

Figure 1 represents in perspective a general aspect of the improved structure for protection glasses.

Figure 2a represents a usual use of the improved structure for protection glasses from a side view with a detail of the falling light beams.

Figure 2b representes a usual use of the improved structure for protection glasses from a front view with the detail of the falling light beams.

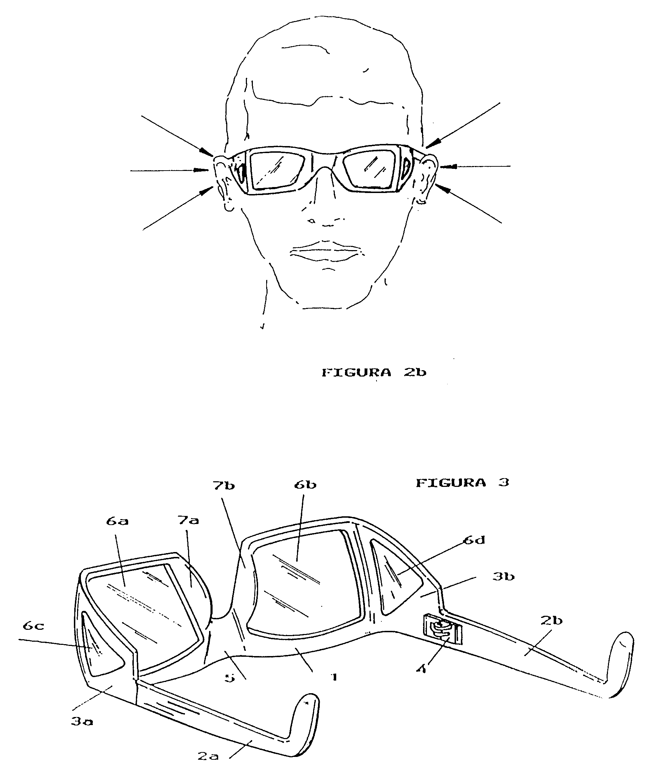

Figure 3 represents a perspective view of the interior zone of the improved structure for protection glasses.

As it can be observed from the referenced figures, the improved structure for protection glasses consists of a main body (1) which contains the essential structure and of two side-arms (2a) and (2b) which are joined to the side side zones (3a) and (3b) of the structure. The joining means between the three bodies is a hinge (4) of suitable size for said structure, which permits for the collapsing of the side-arms (2a) and (2b) from sides (3a) and (3b) with a movement toward the inside angle between the side-arms (2a) and (2b) and the main body (1). The structure of the side-arms (2a) and (2b) is such as the known ones in any conevntional glasses.

On the other hand, the main body has a front structure to which both right and left laterals (3a) and (3b) respectively are joined, forming an assembly made and manufactured in a single mould. One can say that being bridge (5) the central zone of the structure (1) this latter is symmetrical with resepct to said bridge (5). In both left and right zones with respect to the bridge (5) there are two empty spaces surrounded by the structure. Both empty spaces having a necessary filtering element (6a) and (6b), characterised in that the contour thereof is adapted to the empty space and that in the body thereof an optical filter is present which impedes the passage of ultraviolet beams or ultraviolet radiation through said material. The material has a thickness much less than any of the dimensions which intervene in the area defined by its acontour.

The approximate shape of said filtering elements (6a) and (6b) corresponds to a trapezium whose parallel sides are situated on a horizontal coordination the larger horizontal side being at the upper zone and the shorter horizontal side being at the lower zone. In addition, the vertical sides have a slope which defines the union with the two horizontal sides, the interior side having more inclination than that of the exterior, which is practically vertical. One characterisitic of this trapezium is that it has round vertices.

With regard to the lateral zones (3a) and (3b), these are designed in shape as an irregular trapezium containing two of the four vertices at the upper part thereof, a third vertex at the lower part and the fourth vertex at the medium exterior zone having the side which joins the third and the fourth vertices an inclination of approximately 45^{º}. In the interior thereof there is an empty space in order to place therein a filtering element at each side (6c and 6d) left and right, having less dimensions than the filtering elements of the front sides and having the shape of equilateral triangle with the base adapted to the interior contour defining a totally vertical side. The material has a thickness much less than that of any of the dimensions which intervene in the area defiend by its contour. Further, the rounded vertices of the same are adapted to the sahpe of the empty space.

In the interior part of the improved structure there are two protuberances (7a) and (7b) in order to adapt the central bridge (5) to the nose ofthe patient, such that with this mentioned gripping means and the gripping of the contour of the side-arms (2a) and (2b) to the ears of the patient a perfect adaptation at the zone of the face corresponding to the hight of the eyes is achieved.

Furthermore, inside each front empty space conventional graduated cristals may be adapted in order to be placed adjacent to the interior side of the filtering elements by means of a recess made in the contour of the front empty spaces of the structure.
having sufficiently described the nature of the present invention as well as one way of putting it into practice, it only remains to be added that it possible to introduce changes of form, material and disposition of the invention as a whole or in parts of which it is composed, as long as said alterations do not substantially vary the characteristics of the invention which are claimed as follows.

## Claims

1. Improved structure for protection glasses, characterised in the objective of serving for treatment after an ocular intervention which would need the elimination of falling ultraviolet radiation onto the eyes of the patient. Characterised in its structure by composing a front side and two lateral sides which form the main body to which they are adapted by means of hinges dimensioned for the size of the structure, two side-arms for the gripping of the structure on both sides of the face ofthe pateint at the zone corresponding to the ears.

2. Improved structure for protection glasses, according to the previous claim, characterised in that at the front part of the structure there is a bridge for adaptation at the upper zone of the patient's nose, both sides of the structure being symmetrical with respect to said bridge. Said bridge has, at the interior side thereof, two protuberances adaptable to the sides of the nose from the upper zone to the middle zone of the same.

3. Improved structure for protection glasses, according to claim 1, characterised by having in two symmetrical empty zones of the structure at the front part of the same, two filtering elements eliminating ultraviolet radiation such as conventional cristal for glasses which intervene in the whole filed of vision of each eye.

4. Improved structure for protection glasses, according to claim 1, characterised by having at each lateral side, an empty space destined for placing a filtering element eliminating ultraviolet radation each filter intervening in the whole field of lateral vision of each eye.

5. Improved structure for protection glasses, according to claim 1, characterised in that by means of a recess or notch in the contour of each front empty space, graduated cristals may be adapted for the use of the patient in the case of need of the latter, said graduated cristals may be located together with the ultraviolet radiation filtering elements due to the standarised shape of the structure of the invention, siad adding of graduated cristals being on the interior side.
